# EUROPEAN PATENT SPECIFICATION

(11) **EP 3 779 306 B1**
(45) Date of publication and mention of the grant of the patent: **25.01.2023**
(21) Application number: 19776612.4
(22) Date of filing: 22.03.2019
(51) Int. Cl.: F24F 11/70, A61B 5/11, A61B 5/16, F24F 1/008, A61L 9/12, F24F 120/14, F24F 8/50

(54) **AROMA RELEASING SYSTEM**
AROMAFREISETZUNGSSYSTEM
SYSTÈME DE LIBÉRATION D'ARÔME

(30) Priority: 30.03.2018 JP 2018070250
(43) Date of publication of application: 17.02.2021
(73) Proprietor: DAIKIN INDUSTRIES, LTD., Osaka-Shi, Osaka 530-0001 (JP)
(72) Inventor: KITAGAWA, Keita, Osaka 530-8323 (JP); HANDA, Youichi, Osaka 530-8323 (JP)
(74) Representative: Global IP Europe Patentanwaltskanzlei
(86) International application number: PCT/JP2019/012117
(87) International publication number: WO 2019/188791

(56) References cited:
- WO-A1-2017/022157
- WO-A1-2018/058132
- CN-A- 107 023 952
- JP-A- H0 755 226
- JP-A- H05 245 122
- JP-A- H05 302 731
- JP-A- H07 328 079
- JP-A- 2003 038 630
- JP-A- 2006 320 621
- JP-A- 2007 151 933
- JP-A- 2011 122 732
- JP-A- 2017 033 226
- KR-A- 20120 021 914

## Description

### TECHNICAL FIELD

The present disclosure relates to an aroma diffusion system that diffuses an aroma into a target space.

### BACKGROUND ART

Patent Literature 1 (JP 2007-170761 A) discloses a known aroma diffusion system configured to diffuse an aroma according to, for example, an environmental condition such as a temperature or a humidity, a time, an occupation of a user, or information as to whether a user smokes. According to such a system, a diffused aroma makes a space comfortable for a user to a certain degree.

JP 2017 033226 A discloses an aroma diffusion system comprising: an aroma diffusion unit having a plurality of aroma patterns to be diffused, the aroma diffusion unit being configured to diffuse an aroma corresponding to the aroma pattern into a target space; an acquisition unit configured to acquire person information on at least one of an activity and a mood of a person in the target space; a measurement unit configured to measure a temperature and a humidity of the target space; a storage unit including, as information storage regions, a correspondence information database and a pattern database, the correspondence information database storing a list of a combination of the activity or the mood of the person in the target space, the temperature of the target space, and the humidity of the target space, and the aroma pattern, the combination and the aroma pattern being associated with each other in the list, the pattern database storing a list of an aroma type, an aroma diffusion amount, and an aroma diffusion duration set for each of the aroma patterns; and an identification unit configured to identify at least one of the activity and the mood of the person in the target space, based on the person information acquired by the acquisition unit; an aroma determination unit configured to determine the aroma pattern of the aroma diffusion unit based on the information stored in the correspondence information database, a result of the identification by the identification unit, and the temperature of the target space and the humidity of the target space; an aroma diffuser control unit configured to, when the aroma determination unit determines the aroma pattern, read, referring to the pattern database, the aroma type, the aroma diffusion amount, and the aroma diffusion duration corresponding to the aroma pattern determined by the aroma determination unit, and control the aroma diffuser to operate under the read-out conditions to diffuse an aroma corresponding to the determined aroma pattern into the target space.

JP H05 302731 A and JP 2003 038630 A each disclose an aroma diffusion system comprising: an aroma diffusion unit having a plurality of patterns of aromas to be diffused, the aroma diffusion unit being configured to diffuse an aroma corresponding to the pattern into a target space; an acquisition unit configured to acquire person information on at least one of an activity and a mood of a person in the target space; a storage unit stores, as first information, multiple types of activities and/or multiple types of moods, and the patterns, with associating the respective activities and/or the respective moods with the pattern; an identification unit configured to identify at least one of the activity and the mood of the person in the target space, based on the person information acquired by the acquisition unit; and an aroma determination unit configured to determine the pattern of the aroma diffusion unit, based on the first information stored in the storage unit and a result of the identification by the identification unit.

JP H05 245122 A discloses a refreshment device configured to detect a skin potential signal from a human body, determine a degree of fatigue of the human body based on the detected skin potential signal, and diffuse an aroma when the degree of fatigue is high.

### SUMMARY OF THE INVENTION

### <Technical Problem>

However, even under the same condition (even at the same temperature and the same time), whether a user feels a certain aroma pleasant may vary depending on a state (e.g., an activity, a mood) of the user.

Therefore, the aroma diffusion system disclosed in Patent Literature 1 (JP 2007-170761 A) has room for improvement in determination of an aroma to be diffused.

### <Solutions to Problem>

An aroma diffusion system according to the invention is disclosed in claim 1. Claims 2 to 7 disclose further aspects of the invention.

### BRIEF DESCRIPTION OF THE DRAWINGS

FIG. 1 is a schematic configuration diagram of an aroma diffusion system according to an embodiment.
FIG. 2 is a schematic block diagram of the aroma diffusion system illustrated in FIG. 1.
FIG. 3 is a schematic block diagram of a control apparatus of the aroma diffusion system, the control apparatus including a controller of an air conditioning apparatus and a server.
FIG. 4 is a diagram of exemplary information stored in a correspondence information database of a storage unit of the control apparatus illustrated in FIG. 3.
FIG. 5 is a diagram of exemplary aroma types suitable for various person's activities and moods.
FIG. 6 is a diagram of exemplary aroma patterns stored in a pattern database of the storage unit of the control apparatus illustrated in FIG. 3.
FIG. 7 is an exemplary flowchart of aroma control on a target space by the control apparatus illustrated in FIG. 3.

### DESCRIPTION OF EMBODIMENTS

A description will be given of an aroma diffusion system 1 according to an embodiment of the invention.

### (1) General Configuration

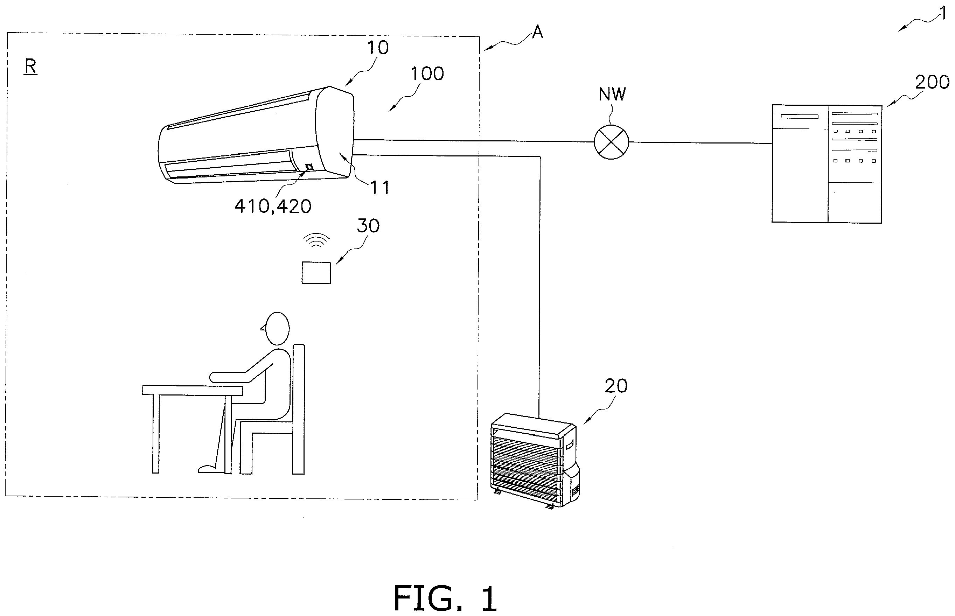

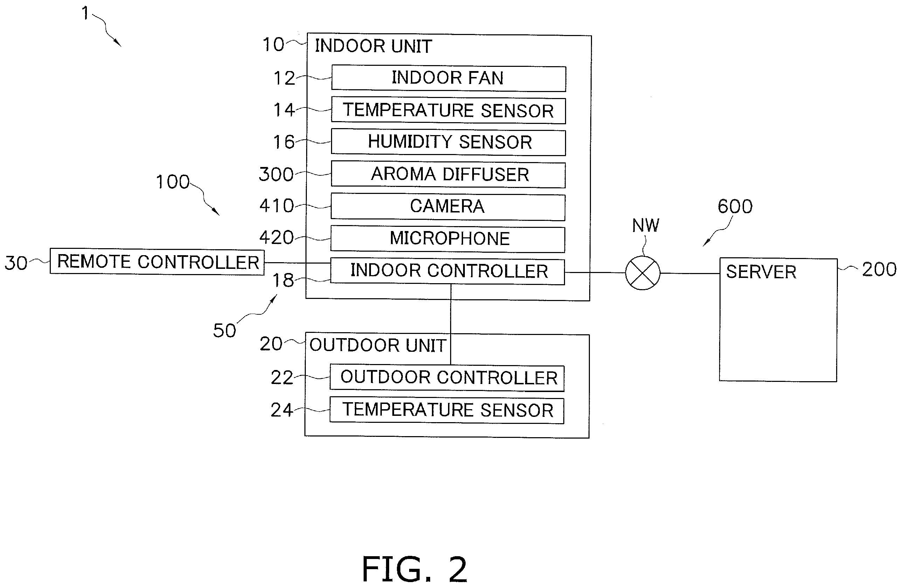

With reference to FIGS. 1 and 2, a description will be given of an outline of the aroma diffusion system 1. FIG. 1 is a schematic configuration diagram of the aroma diffusion system 1. FIG. 2 is a schematic block diagram of the aroma diffusion system 1.

The aroma diffusion system 1 is configured to diffuse an aroma into a target space R. Specifically, the aroma diffusion system 1 is configured to identify an activity and a mood of a person in the target space R and to diffuse an aroma into the target space on a condition based on a result of the determination (i.e., in accordance with a pattern of an aroma determined based on a result of the identification).

In this embodiment of the invention, the target space R is a room of a house in which a person lives. However, the target space R is not limited thereto. For example, the target space R may be a space in an office or a factory. Alternatively, the target space R may be a space in a commercial facility.

In embodiment of the invention, a person's activity means what kind of activity a person is performing. In this embodiment, examples of a person's activity (i.e., a person's activity to be identified by the aroma diffusion system 1) may include, but not limited to, a state in which a person is sleeping (a sleeping state), a state in which a person awakes from sleep (a rising state), a state in which a person is doing some work (a working state), a state in which a person has returned home and entered a room, and a state in which a person is fidgeting. The aroma diffusion system 1 may identify more details of the working state. For example, the aroma diffusion system 1 may distinguish and identify a state in which a person is using a personal computer or doing some writing while sitting, a state in which a person is doing some work such as cleaning or cooking while standing up, a state in which a person is holding a conversation with someone, a state in which a person is holding a conversation with someone by telephone, and others. The listed person's activities to be identified by the aroma diffusion system 1 are merely examples. The aroma diffusion system 1 may identify any person's activity in addition to the listed person's activities. Alternatively, the aroma diffusion system 1 does not necessarily identify some of or all of the listed person's activities.

In this embodiment of the invention, a person's mood means a person's mind and body state such as pleasantness or unpleasantness. In this embodiment, examples of moods (i.e., person's moods to be identified by the aroma diffusion system 1) may include, but not limited to, a drowsy state, a tired state, a grumpy state, and a gloomy state. Examples of the person's mood to be identified by the aroma diffusion system 1 may also include, but not limited to, a tense state and a relaxed state. The listed person's moods to be identified by the aroma diffusion system 1 are merely examples. The aroma diffusion system 1 may identify any person's mood in addition to the listed person's moods. Alternatively, the aroma diffusion system 1 does not necessarily identify some of or all of the listed person's moods.

In this embodiment of the invention, an aroma pattern is determined by a combination of an aroma type, an aroma diffusion amount (i.e., the intensity of an aroma), and an aroma diffusion duration. In other words, a sentence, an aroma pattern (referred to as a pattern X) is different from another aroma pattern (referred to as a pattern Y), means that the pattern X is different in at least one of the above mentioned elements from the pattern Y. A specific description on the aroma pattern will be given later.

A description will be given of appliances that constitute the aroma diffusion system 1.

The aroma diffusion system 1 mainly includes an air conditioning apparatus 100 including an indoor unit 10 equipped with an aroma diffuser 300 and a server 200 connected to the air conditioning apparatus 100, specifically a controller 50 (to be described later) of the air conditioning apparatus 100 via a network NW so as to establish communications with the controller 50 (see FIGS. 1 and 2). The network NW used herein is the Internet; however, the network NW may be, for example, another wide area network or a local area network.

The air conditioning apparatus 100 is installed in a building (a house A) (see FIG. 1). The air conditioning apparatus 100 performs air conditioning in a room (a target space R) of the house A where the indoor unit 10 is placed (see FIG. 1). The aroma diffuser 300 in the indoor unit 10 of the air conditioning apparatus 100 diffuses an aroma into the target space R.

The server 200 is a computer configured to execute various processes. The server 200 may be installed in any place. The server 200 is typically installed in a place different from the house A.

In FIG. 1, the server 200 is connected only to, but not limited to, the air conditioning apparatus 100 installed in the house A, via the network NW. The server 200 may be connected to air conditioning apparatuses in multiple houses (or buildings other than houses) via the network NW. In other words, the server 200 may constitute the aroma diffusion system 1 in conjunction with the air conditioning apparatus 100 installed in the house A, and may also constitute another aroma diffusion system in conjunction with an air conditioning apparatus installed in a different building. For the sake of simplifying the description, it is assumed herein that the server 200 is connected only to the air conditioning apparatus 100 in the house A via the network NW.

For the sake of simplifying the description, it is also assumed herein that the house A has one room (the target space R), and one air conditioning apparatus 100 is installed in the house A to achieve air conditioning in the target space R. However, the number of rooms is not limited thereto, and the installation of the air conditioning apparatus 100 is also not limited thereto. For example, the house A may have a plurality of rooms, and air conditioning apparatuses may be respectively installed in the rooms. For the sake of simplifying the description, it is also assumed herein that the air conditioning apparatus 100 includes one indoor unit 10 (including the aroma diffuser 300) to be placed in the target space R. Alternatively, the air conditioning apparatus 100 may include at least two indoor units 10 to be placed in the target space R.

The server 200 is one computer, but is not limited to a computer. The server 200 may include a plurality of computers installed in the same place or respectively installed in different places, and connected to one another via a network.

### (2) Specific Configuration

With reference to FIGS. 1 to 6, a description will be further given of the air conditioning apparatus 100 and the server 200. In the following, a specific description will be given of a control apparatus 600 that includes the controller 50 of the air conditioning apparatus 100 and the server 200.

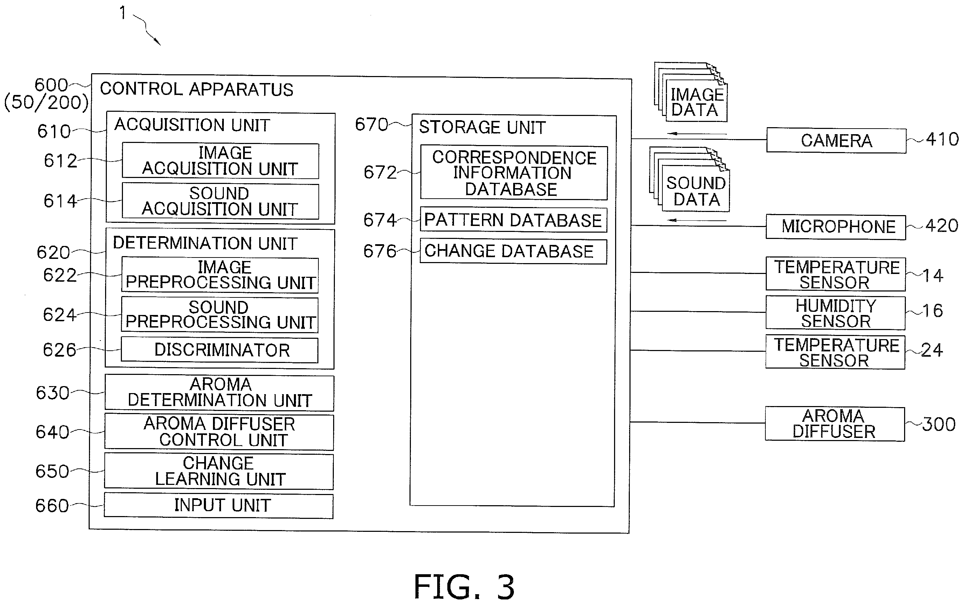

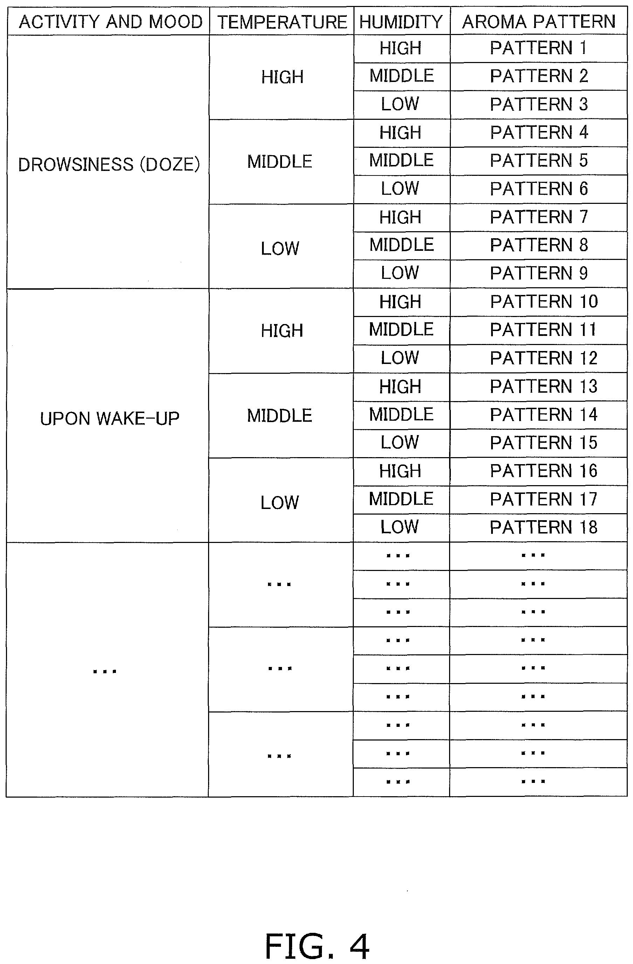

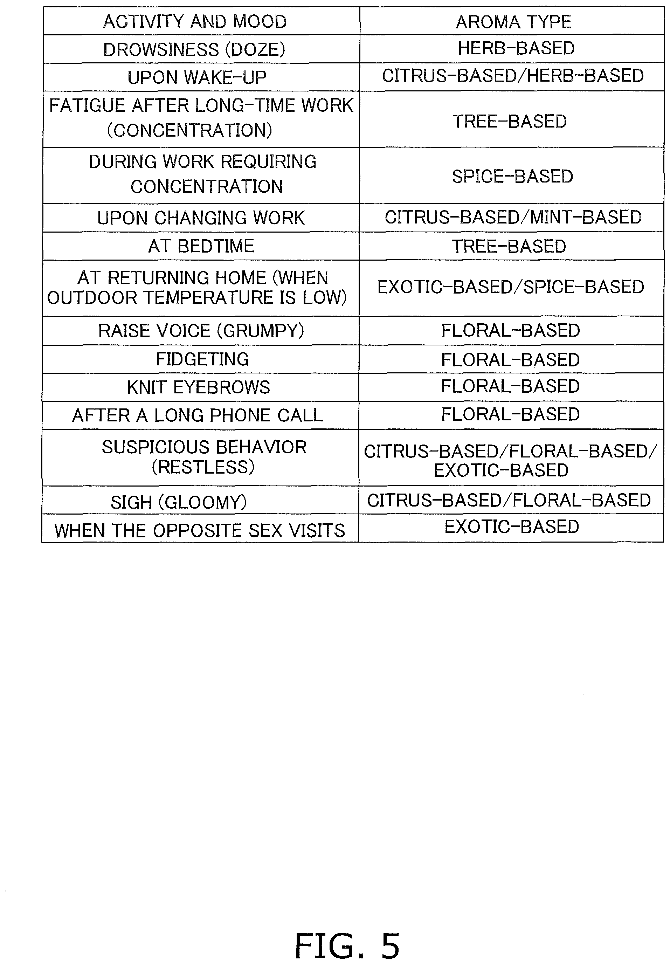

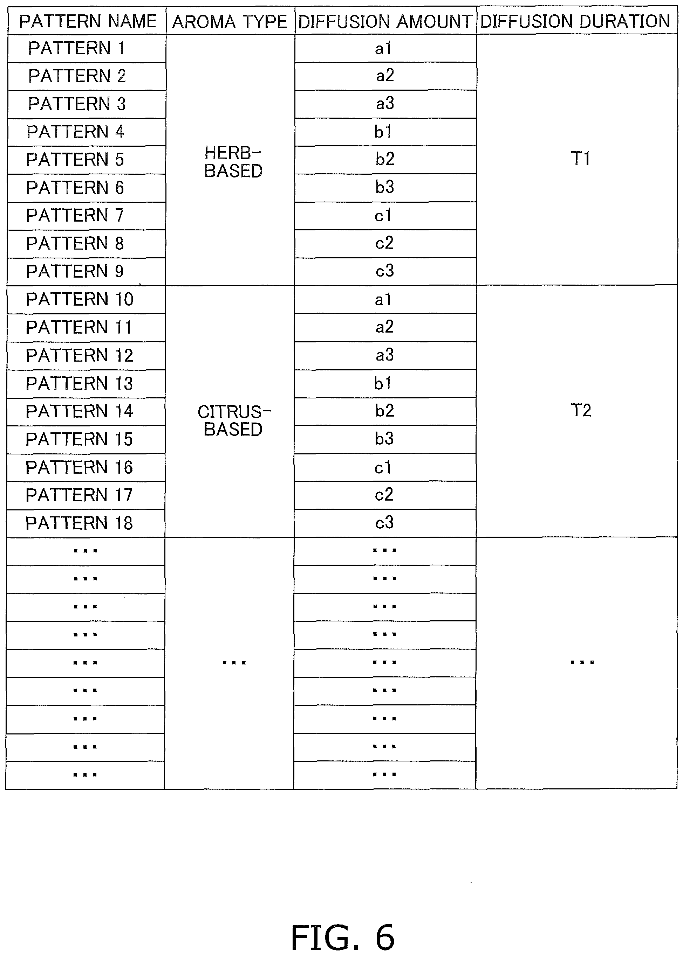

FIG. 3 is a schematic block diagram of the control apparatus 600 including the controller 50 of the air conditioning apparatus 100 and the server 200. FIG. 4 is a diagram of exemplary information stored in a correspondence information database 672 of a later-described storage unit 670 of the control apparatus 600. FIG. 5 is a diagram of exemplary aroma types suitable for various person's activities and moods. FIG. 6 is a diagram of exemplary aroma patterns stored in a pattern database 674 of the storage unit 670 of the control apparatus 600.

### (2-1) Air Conditioning Apparatus

The air conditioning apparatus 100 performs a cooling operation (including a dehumidifying operation) and a heating operation in the target space R where the indoor unit 10 is placed. The air conditioning apparatus 100 performs, a cooling operation and a heating operation using a vapor compression refrigeration cycle. However, the air conditioning apparatus 100 is not necessarily an apparatus that performs a cooling operation and a heating operation. For example, the air conditioning apparatus may be a cooling-only apparatus.

The air conditioning apparatus 100 mainly includes the indoor unit 10, an outdoor unit 20, a connection pipe (not illustrated) connecting the indoor unit 10 to the outdoor unit 20, and a remote controller 30 (see FIGS. 1 and 2). The indoor unit 10 illustrated in FIG. 1 is designed to be hung on a wall. Alternatively, the air conditioning apparatus 100 may include an indoor unit of any type such as an indoor unit designed to be mounted on a ceiling (e.g., an indoor unit designed to be embedded in a ceiling, an indoor unit designed to be suspended from a ceiling) or an indoor unit designed to be placed on a floor.

In the air conditioning apparatus 100, the indoor unit 10 is connected to the outdoor unit 20 via the connection pipe. Thereby, an indoor heat exchanger (not illustrated) of the indoor unit 10 as well as a compressor, an outdoor heat exchanger, an expansion valve, and the like (not illustrated) of the outdoor unit 20 are connected via pipes to form a refrigerant circuit. The air conditioning apparatus 100 causes a refrigerant to circulate through the refrigerant circuit such that the refrigerant circulating through the refrigerant circuit exchanges heat with air in the target space R. The air conditioning apparatus 100 thus achieves a cooling operation or a heating operation in the target space R where the indoor unit 10 is placed. Specifically, the air conditioning apparatus 100 achieves a cooling operation or a heating operation in the target space R in such a manner that an indoor fan 12 (see FIG. 2) of the indoor unit 10 takes air in the target space R into a housing 11 and supplies it to the indoor heat exchanger (see FIG. 1) of the indoor unit 10, and then blows out the air being subjected to heat exchange with the refrigerant flowing through the indoor heat exchanger, toward the target space R. The operation principle of the air conditioning apparatus 100 using the vapor compression refrigeration cycle is common knowledge; therefore, the description thereof will not be given here.

The indoor unit 10 includes the indoor heat exchanger (not illustrated), the indoor fan 12, the housing 11 accommodating the indoor heat exchanger and the indoor fan 12 therein, a temperature sensor 14 that measures a temperature of the target space R, a humidity sensor 16 that measures a humidity of the target space R, and an indoor controller 18 (see FIGS. 1 and 2). The indoor controller 18 is, for example, a micro control unit (MCU) mainly including a central processing unit (CPU), a memory, an input-output port, and the like. The indoor unit 10 is equipped with a camera 410 and a microphone 420 in addition to the aroma diffuser 300. The aroma diffuser 300, the camera 410, and the microphone 420 will be described later.

The outdoor unit 20 mainly includes a compressor, an outdoor heat exchanger, an expansion valve, and an outdoor fan (not illustrated), a temperature sensor 24 that measures a temperature of the outside where the outdoor unit 20 is placed, and an outdoor controller 22. The outdoor controller 22 is, for example, an MCU mainly including a CPU, a memory, an input-output port, and the like.

The remote controller 30 is an appliance that accepts the inputting of various instructions and various kinds of information from a user of the air conditioning apparatus 100. The remote controller 30 includes an MCU mainly including a CPU, a memory, an input-output port, and the like. The remote controller 30 may include a display unit (not illustrated) configured to display thereon, for example, a current operating state of the air conditioning apparatus 100.

The indoor controller 18, the outdoor controller 22, and the remote controller 30 are connected to establish mutual communications, and constitute the controller 50 as a whole. The controller 50 controls the operation of the air conditioning apparatus 100. The controller 50 executes a program stored in the memory, thereby controlling the operation of an appliance for air conditioning in the air conditioning apparatus 100, based on instructions input to the remote controller 30 by the user and measured values of sensors in the respective units of the air conditioning apparatus 100. The sensors in the respective units of the air conditioning apparatus 100 include the temperature sensor 14, the humidity sensor 16, and the temperature sensor 24. Examples of the appliance for air conditioning in the air conditioning apparatus 100 whose operation is controlled by the controller 50 include: the compressor, expansion valve, and outdoor fan (not illustrated) of the outdoor unit 20; and the indoor fan 12 of the indoor unit 10. The control by the controller 50 on the operation of the appliance for air conditioning is typically known; therefore, the description thereof will not be given here.

The controller 50 is connected to the network NW via a modem or an optical network unit, a router, and others (not illustrated), so that the controller 50 is connected to the server 200 so as to establish communications with the server 200. The controller 50 constitutes the control apparatus 600 in conjunction with the server 200 (see FIG. 3).

The control apparatus 600 controls the operation of the aroma diffuser 300 in the aroma diffusion system 1. In summary, the control apparatus 600 identifies the activity and the mood of the person in the target space R, determines a pattern (an aroma pattern) of an aroma to be diffused from the aroma diffuser 300, based on a result of the identification, and controls the operation of the aroma diffuser 300 such that the aroma diffuser 300 diffuses the aroma corresponding to the determined pattern. The control apparatus 600 will be described in detail later.

### (2-1-1) Aroma Diffuser

The aroma diffuser 300 is an example of an aroma diffusion unit having a plurality of patterns (aroma patterns) of aromas to be diffused. The aroma diffusion unit diffuses an aroma corresponding to the pattern into the target space R.

A state in which "the aroma diffuser 300 has a plurality of patterns of aromas to be diffused" used herein does not necessarily mean that the aroma diffuser 300 previously has an aroma pattern (in other words, an operation pattern). The state in which "the aroma diffuser 300 has a plurality of patterns of aromas to be diffused" involves a state in which the aroma diffuser 300 is capable of diffusing an aroma by changing its operation pattern in accordance with an instruction from the control apparatus 600.

The aroma diffuser 300 includes a plurality of aroma reservoirs (not illustrated). The aroma reservoirs respectively store aromas that are different in types from one another. Examples of the aromas stored in the aroma reservoirs may include a herb-based aroma, a citrus-based aroma, a tree-based aroma, a spice-based aroma, a mint-based aroma, an exotic-based aroma, and a floral-based aroma. However, the aromas stored in the aroma reservoirs are not limited to the listed aromas. The aromas stored in the aroma reservoirs may include any aromas in addition to the listed aromas. Alternatively, the aromas stored in the aroma reservoirs may not include some of or all of the aromas.

The aroma diffuser 300 diffuses an aroma into the target space R in accordance with an aroma pattern instructed by the control apparatus 600. The aroma pattern as used herein is defined based on three elements, that is, an aroma type, an aroma diffusion amount per unit time (i.e., an aroma diffusion amount), and an aroma diffusion duration (see FIG. 6). In other words, the aroma diffuser 300 is capable of controlling a type of an aroma (a perfume) to be diffused, an amount of the aroma diffused per unit time (hereinafter, simply referred to as an aroma diffusion amount), and a duration at which the aroma is diffused.

The aroma pattern as used herein is merely an example, and is not limited to thereto. For example, the aroma pattern may be defined based on one of the three elements or may be defined based on two of the three elements. In other words, the aroma diffuser 300 may be configured to change, for example, only the aroma type. Alternatively, the aroma diffuser 300 may be configured to change, for example, only the aroma type and the aroma diffusion amount. Examples of the elements for defining the aroma pattern may include a timing of diffusing an aroma. The aroma diffuser 300 may also be configured to diffuse two or more aromas in the aroma reservoirs at the same time. Examples of the elements for defining the aroma pattern may also include a mixing ratio of aromas (e.g., a ratio between an amount of an aroma of a type "a" and an amount of an aroma of a type "b", the aromas being diffused at the same time).

For example, the aroma diffuser 300 is disposed in the housing 11 (see FIG. 1) of the indoor unit 10 (the aroma diffuser 300 is not illustrated in FIG. 1). For example, the aroma diffuser 300 is disposed at a position where a person is able to perform various kinds of maintenance by opening a front panel of the housing 11 of the indoor unit 10. The position of the aroma diffuser 300 is not limited to the inside of the housing 11 of the indoor unit 10. For example, the aroma diffuser 300 may be disposed on an outer surface of the housing 11. Alternatively, the aroma diffuser 300 may be disposed at a position adjacent to the housing 11.

Preferably, the aroma diffuser 300 diffuses an aroma to a flow path of air generated by the indoor fan 12. According to this configuration, the aroma diffesed from the aroma diffuser 300 readily spreads throughout the target space R where air flows.

### (2-1-2) Camera

The camera 410 is an apparatus that captures an image of the target space R. The camera 410 is particularly an apparatus configured to capture an image of the person in the target space R. Preferably, the camera 410 is an apparatus that captures a moving image. However, the camera 410 may be an apparatus that captures a still image in a case where a still image is used for identifying a person's activity and a person's mood as described later. Preferably, the camera 410 captures a color image. In a case where, for example, hue information is not used, the camera 410 may be a decive that captures a monochrome image. The camera 410 is connected to the control apparatus 600 so as to establish communications with the control apparatus 600. The camera 410 transmits a captured image (image data) to the control apparatus 600.

The camera 410 is disposed on, for example, an outer surface of a front panel of the housing 11 of the indoor unit 10 (see FIG. 1). The camera 410 is not necessarily disposed on the outer surface of the front panel of the housing 11. The position of the camera 410 may be appropriately selected. For example, the camera 410 may be disposed on a lower surface of the housing 11. Alternatively, the camera 410 may be disposed, for example, at a position adjacent to the housing 11 rather than on the housing 11. Preferably, the camera 410 is disposed at a position where the camera 410 captures an image of a wide area of the target space R with ease.

According to another embodiment, the camera 410 may be an apparatus provided independently of the indoor unit 10 and disposed at a position distant from the housing 11. Also in this case, preferably, the camera 410 is connected to the control apparatus 600 so as to establish communications with the control apparatus 600, and is configured to transmit image data to the control apparatus 600.

In this embodiment, the number of cameras 410 is one. However, the number of cameras 410 is not limited to one. The indoor unit 10 may be equipped with a plurality of cameras 410.

### (2-1-3) Microphone

The microphone 420 is an apparatus that convert a sound of the target space R into an electric signal, thereby acquiring the sound in the form of the electric signal. The microphone 420 is particularly an apparatus configured to acquire a sound which a person in the target space R makes. Examples of the sound which the person in the target space R makes may include, but not limited to, a voice, a sigh, a yawn, breathing, and noise due to fidgeting. The microphone 420 preferably includes a plurality of microphone elements in order to identify a direction from which a sound is generated (e.g., a direction from which a voice is originated). The microphone 420 is connected to the control apparatus 600 so as to establish communications with the control apparatus 600. The microphone 420 transmits an acquired sound data to the control apparatus 600.

The microphone 420 is disposed on, for example, the outer surface of the front panel of the housing 11 of the indoor unit 10 at a position near the camera 410 (see FIG. 1). The microphone 420 is not necessarily disposed on the outer surface of the front panel of the housing 11. The position of the microphone 420 may be appropriately selected. For example, the microphone 420 may be disposed at a position distant from the camera 410. Alternatively, the microphone 420 may be disposed on the lower surface of the housing 11. Alternatively, the microphone 420 may be disposed, for example, at a position adjacent to the housing 11 rather than on the housing 11. Preferably, the microphone 420 is disposed at a position where the microphone 420 acquires a sound in the target space R with ease.

According to another embodiment, the microphone 420 may be an apparatus provided independently of the indoor unit 10 and disposed at a position distant from the housing 11. Also in this case, preferably, the microphone 420 is connected to the control apparatus 600 so as to establish communications with the control apparatus 600, and is configured to transmit image data to the control apparatus 600.

In this embodiment, the microphone 420 (a group of microphone elements) is disposed on a single place. Alternatively, the microphone elements may be respectively disposed on different places.

### (2-2) Server

The server 200 is a computer connected to the controller 50 of the air conditioning apparatus 100 via the network NW so as to establish communications with the controller 50 and constituting the control apparatus 600 in conjunction with the controller 50. The server 200 mainly includes a CPU, a random access memory (RAM), a read only memory (ROM), and an external storage device such as a hard disk. The server 200 executes various programs stored in the ROM, the external storage device, and the like, thereby executing various processes as the control apparatus 600 in conjunction with the controller 50.

### (2-3) Control Apparatus

The control apparatus 600 includes the controller 50 of the air conditioning apparatus 100 and the server 200. The controller 50 and the server 200 are connected via the network NW so as to establish communications with each other.

The control apparatus 600 includes, as its functional unit, an acquisition unit 610, an identification unit 620, an aroma determination unit 630, an aroma diffuser control unit 640, a change learning unit 650, an input unit 660, and a storage unit 670 (see FIG. 3).

In this embodiment, for example, the server 200 has functions as the acquisition unit 610 and the identification unit 620, the remote controller 30 of the controller 50 has a function as the input unit 660, and the indoor controller 18 of the controller 50 has functions as the aroma determination unit 630, the aroma diffuser control unit 640, and the change learning unit 650. The controller 50 includes the correspondence information database 672, the pattern database 674, and a change database 676 of the storage unit 670. The control apparatus 600 is merely required to function as the acquisition unit 610, the identification unit 620, the aroma determination unit 630, the aroma diffuser control unit 640, the change learning unit 650, the input unit 660, and the storage unit 670 as a whole. These functions may be appropriately allocated to the respective units of the controller 50 and server 200.

### (2-3-1) Acquisition Unit

The acquisition unit 610 acquires person information on at least one of the activity and the mood of the person in the target space R. Specifically, the acquisition unit 610 acquires, as person information, image data transmitted from the camera 410 and sound data transmitted from the microphone 420.

The acquisition unit 610 includes an image acquisition unit 612 that acquires an image (image data) of the target space R transmitted from the camera 410. The acquisition unit 610 also includes a sound acquisition unit 614 that acquires a sound (sound data) of the target space R transmitted from the microphone 420, particularly a sound which a person in the target space R makes. The image and sound of the target space R acquired by the acquisition unit 610 are stored in the storage unit 670, and are used for, for example, processes to be executed by the identification unit 620 as will be described later.

### (2-3-2) Identification Unit

The identification unit 620 identifies the activity and the mood of the person in the target space R based on the person information (e.g., image data and sound data) acquired by the acquisition unit 610. For example, the identification unit 620 identifies multiple activities (e.g., the sleeping state, the rising state, the working state, the state in which a person has returned home and entered a room, the state in which a person is fidgeting) of the person in the target space, based on the person information acquired by the acquisition unit 610. For example, the identification unit 620 also identifies multiple moods (e.g., the drowsy state, the tired state, the grumpy state, the gloomy state) of the person in the target space, based on the person information acquired by the acquisition unit 610. The identification unit 620 may identify as "not applicable" when the person information acquired by the acquisition unit 610 does not apply to any of the activity and the mood of the person to be subjected to identification.

The identification unit 620 includes an image preprocessing unit 622, a sound preprocessing unit 624, and a discriminator 626 (see FIG. 3).

The identification unit 620 identifies the activity and the mood of the person in a target space based on person information (e.g., image data and sound data) acquired by the acquisition unit 610, using the learned discriminator 626 (the learning will be described later).

As preprocessing for image data in person information acquired by the acquisition unit 610, the image preprocessing unit 622 extracts image information (at least one of the person whole-body region image and the person partial-body region image to be described later) for the identification unit 620 to identify the activity and the mood of the person in a target space.

As preprocessing for sound data in person information acquired by the acquisition unit 610, the sound preprocessing unit 624 detects a target sound section by discriminating, as to sound data containing a target sound and noise, a section where the target sound is present from the rest. The sound preprocessing unit 624 then extracts the section where the target sound is present from the sound data. The target sound as used herein includes human voices (involving voices devoid of meaning, such as a sigh and a yawn), a sound generated when a person is fidgeting, and others. In addition, the sound preprocessing unit 624 extracts a sound feature amount from the target sound.

### (2-3-2-1) Image Preprocessing Unit

For example, the image preprocessing unit 622 extracts the person whole-body region image from image data acquired by the acquisition unit 610. For example, the image preprocessing unit 622 extracts a moving object region in which an image changes (a region in which it is presumed that a moving object is present) using a differential image between a background image stored in the storage unit 670 in advance and image data or a differential image between an image captured at a certain point in time and an image captured earlier than the certain point by a predetermined time. When a size and/or an aspect ratio of the moving object region (or a group of moving object regions presumed as those obtained by capturing images of the same object) matches to conditions of a human body, the image preprocessing unit 622 defines this region as a person region and extracts an image of this region as the person whole-body region image. The image preprocessing unit 622 obtains time-series data on person whole-body region images through the processing described above. When a distance between the person whole-body region image captured at a certain point in time and the person whole-body region image captured earlier than the person whole-body region image by a predetermined time falls within a predetermined distance, the image preprocessing unit 622 correlates the person whole-body region images with each other as the images of the same person, thereby enabling a tracking process.

The method of extracting the person whole-body region image is not limited to the method described above. For example, the person whole-body region image may be extracted through a matching process using a person template. In a case where the image is a color image, alternatively, the person whole-body region image may be extracted using, for example, color information (e.g., a color of a skin). Alternatively, the person whole-body region image may also be extracted using information obtained by an appliance different from the camera 410 (e.g., an infrared image captured by an infrared camera). Alternatively, the person whole-body region image may be extracted by any other publicly known methods. The method of the tracking process is not limited to the method described above. For example, well-known methods may be applied to the tracking process.

In addition to or in place of the process of extracting the person whole-body region image from image data, the image preprocessing unit 622 may extract, as the person partial-body region image, a partial image of the person in the target space R (e.g., an image of only a face, an image of only an arm or a leg) from the image data acquired by the acquisition unit 610. The partial region of the person in the image may be extracted by various publicly known methods.

A type of an image to be extracted by the image preprocessing unit 622 may be determined in accordance with what input a later described discriminator 626 uses to learn about an identification of the activity and the mood of the person in the target space R.

### (2-3-2-2) Sound Preprocessing Unit

The sound preprocessing unit 624 detects a target sound section by discriminating, as to sound data containing a target sound and noise, a section where the target sound is present from the rest, and then to extract the section where the target sound is present from the sound data. For example, in a case where a volume equal to or more than a predetermined volume lasts for a predetermined time or more in sound data, the sound preprocessing unit 624 detects this section as a target sound section. However, the method of detecting the target sound section is not limited thereto, and publicly known various methods may be used.

In addition, the sound preprocessing unit 624 extracts a sound feature amount from the target sound while referring to, for example, an acoustic model (not illustrated). Examples of the sound feature amount may include, but not limited to, a volume, a tempo, a tone, and the meaning of a voice. The sound preprocessing unit 624 also refers to a word pronunciation model, a language model, and the like (not illustrated) in addition to the acoustic model, converts a voice into a text, and then recognizes the meaning of the voice. Publicly known various methods may be used for the voice recognition by the sound preprocessing unit 624.

### (2-3-2-3) Discriminator

The discriminator 626 performs a learning process so that the discriminator 626 can output the activity or the mood of the person as a result of determination when receiving at least one of information on an image (particularly at least one of the person whole-body region image and the person partial-body region image) or information on a sound (particularly a sound feature amount). In this embodiment, the discriminator 626 performs a learning process based on machine learning. The machine learning means a technique and a method, which are not rule-based, for a computer to learn about given information and autonomously find rules for determination (even when rules and the like for determination are not given to the computer in advance).

In a case where the discriminator 626 performs discrimination for different inputs, the discriminator 626 may include a plurality of the discriminators. For example, in a case where the discriminator 626 determines a certain activity and a certain mood of a person from only information on an image, determines another activity and another mood of the person from only information on a sound, and determines still another activity and still another mood of the person from information on an image and information on a sound, the discriminator 626 may include a plurality of the discriminators that performs discrimination for different inputs, respectively.

The discriminator 626 has already learned based on, for example, supervised learning. The supervised learning means a method of machine learning for causing the discriminator 626 to learn about training data in which an input is associated with an output as a correct answer (e.g., a person's activity, a person's mood) corresponding to the input.

For example, the discriminator 626 during the learning receives, as training data, multiple pieces of data in which images of a person are associated with activities or moods of the person at the time when the images are captured. The image of the person as used herein may be an entire image of the person (e.g., an image corresponding to the person whole-body region image) or a partial image of the person (an image corresponding to the person partial-body region image). An image usable for identifying at least one of the activity and the mood of the person may be selected as the image of the person. The image of the person as used herein may be a moving image or a still image. In addition, the discriminator 626 during the learning receives, as training data, multiple pieces of data in which sound feature amounts of sounds which various people make are associated with activities or moods of the people at the time when the people make the sounds. In addition to or in place of these pieces of data, the discriminator 626 during the learning may receive, as training data, multiple pieces of data in which images of various people and sound feature amounts of sounds which the people make at the time when the images are captured are associated with activities or moods of the people at the time when the images are captured.

In a case of identifying an activity and a mood of only a certain individual, the discriminator 626 during the learning may receive training data in which images of the individual and sounds which the individual makes are associated with activities or moods of the individual. In order to make the discriminator 626 versatile, preferably, the discriminator 626 during the learning receives training data in which images of various people and sounds which the people make are associated with activities or moods of the people.

A learning algorithm used in the discriminator 626 is, for example, a neural network (including deep learning). Alternatively, other publicly known machine learning algorithms (e.g., a support vector machine) may be used. In the case where the discriminator 626 includes the plurality of discriminators for discriminating different inputs as described above or in a case where a process as will be described later is divided into multiple processes and different discriminators respectively execute the divided processes, different learning algorithms may be used for the learning on the respective discriminators.

When the identification unit 620 receives at least one of the person whole-body region image, the person partial-body region image extracted by the image preprocessing unit 622, and a sound feature amount extracted by the sound preprocessing unit 624, the identification unit 620 identifies the activity or the mood of the person in the target space R using the learned discriminator 626. In other words, the identification unit 620 identifies the activity or the mood of the person in the target space R using the learned discriminator 626, based on, for example, the person whole-body region image extracted by the image preprocessing unit 622 (i.e., based on the whole-body motion of the person in the target space R being acquired by the image acquisition unit 612). For example, the identification unit 620 identifies the activity or the mood of the person in the target space R using the learned discriminator 626, based on a change in posture of the person in the target space R being acquired by the image acquisition unit 612. In addition, the identification unit 620 identifies the activity or the mood of the person in the target space R using the learned discriminator 626, based on, for example, the person partial-body region image extracted by the image preprocessing unit 622 (i.e., based on the partial-body motion of the person in the target space R being acquired by the image acquisition unit 612). For example, the identification unit 620 identifies the activity or the mood of the person in the target space R using the learned discriminator 626, based on movements of facial muscles (e.g., muscles between eyebrows, muscles around a mouth) in the target space R or movements of muscles of an arm or a leg in the target space R being acquired by the image acquisition unit 612. The identification unit 620 may identify the activity or the mood of the person in the target space R using the learned discriminator 626, based on not only the whole-body or partial-body motion of the person in the target space R being acquired by the image acquisition unit 612, but also instantaneous states (e.g., a pose of a person, a facial expression, wrinkles in a face) and a hue or a change in the hue (e.g., a blush on the face). In addition, the identification unit 620 identifies the activity or the mood of the person in the target space R using the learned discriminator 626, based on, for example, a sound feature amount extracted by the image preprocessing unit 622 (i.e., based on a sound which the person in the target space R makes, the sound being acquired by the sound acquisition unit 614).

The identification unit 620 may identify the activity or the mood of the person in the target space R, based on any information in addition to at least one of the person whole-body region image and the person partial-body region image extracted by the image preprocessing unit 622 and a sound feature amount extracted by the sound preprocessing unit 624.

For example, the identification unit 620 may identify a state in which a person has entered the target space R (i.e., a state in which a person has returned to the house A), using, for example, the person whole-body region image extracted by the image preprocessing unit 622, and may further identify a state in which a person who had been in a cold space has entered the target space R, using information on a temperature of the outside measured by the temperature sensor 24.

### (2-3-3) Aroma Determination Unit

The aroma determination unit 630 determines an aroma pattern of the aroma diffuser 300 based on a result of identification (the activity or the mood of the person in the target space R) by the identification unit 620. The aroma determination unit 630 also determines the aroma pattern of the aroma diffuser 300 based on at least one of a result of measurement on the temperature of the target space R detected by the temperature sensor 14 and a result of measurement on the humidity of the target space R detected by the humidity sensor 16. The aroma determination unit 630 determines the aroma pattern of the aroma diffuser 300 based on the activity or the mood of the person in the target space R, the temperature of the target space R, and the humidity of the target space R.

Specifically, the aroma determination unit 630 determines the aroma pattern of the aroma diffuser 300, based on information (to be described later) stored in the correspondence information database 672 of the storage unit 670. The information stored in the correspondence information database 672 is a list of a combination of the activity or the mood of the person in the target space R, the temperature of the target space R, and the humidity of the target space R, and the aroma pattern of the aroma diffuser 300. In the list, the combination and the aroma pattern are associated with each other (see FIG. 4). In the list illustrated in FIG. 4, the temperature and the humidity are each classified into three levels of "high", "middle", and "low" using predetermined threshold values, and aroma patterns are prepared for each of the activity and the mood of the person in the target space R, and for a combination of a level of the temperature and a level of the humidity.

### (2-3-4) Aroma Diffuser Control Unit

When the aroma determination unit 630 determines the aroma pattern, the aroma diffuser control unit 640 controls the aroma diffuser 300 such that the aroma diffuser 300 diffuses an aroma corresponding to the determined aroma pattern into the target space R.

Specifically, when the aroma determination unit 630 determines the aroma pattern, the aroma diffuser control unit 640 refers to the pattern database 674 of the storage unit 670. The pattern database 674 stores a list of an aroma type, an aroma diffusion amount (i.e., an aroma diffusion amount per unit time), and an aroma diffusion duration set for each aroma pattern (see FIG. 6). The aroma diffuser control unit 640 reads an aroma type, an aroma diffusion amount, and an aroma diffusion duration corresponding to the aroma pattern determined by the aroma determination unit 630 and the controls the aroma diffuser 300 such that the aroma diffuser 300 operates under the read out conditions.

The aroma diffuser control unit 640 may control the aroma diffuser 300 such that the aroma diffuser 300 diffuses an aroma only during the operation of the air conditioning apparatus 100.

Alternatively, the aroma diffuser control unit 640 may control the aroma diffuser 300 such that the aroma diffuser 300 diffuses an aroma even when the air conditioning apparatus 100 is not operated. In the case where the aroma diffuser control unit 640 causes the aroma diffuser 300 to diffuse an aroma while the air conditioning apparatus 100 is not operated, the aroma diffuser control unit 640 may issue a request to operate the indoor fan 12 to the controller 50 of the air conditioning apparatus 100.

### (2-3-5) Change Learning Unit

The change learning unit 650 learns about a change in at least one of the activity and the mood of the person before and after the aroma diffuser 300 diffuses the aroma corresponding to the aroma pattern. Specifically, the change learning unit 650 associates the aroma pattern determined by the aroma determination unit 630 with a change in at least one of the activity and the mood of the person before and after the aroma diffuser 300 diffuses the aroma corresponding to the aroma pattern, and then stores the associated information in the change database 676 of the storage unit 670.

More specifically, when the aroma determination unit 630 determines the aroma pattern, the change learning unit 650 stores the activity or the mood of the person in the target space R, which was used for determination of the aroma pattern by the aroma determination unit 630, as the activity or the mood of the person in the target space R before the aroma diffuser 300 diffuses the aroma into the target space R, in the change database 676 of the storage unit 670. After the aroma diffuser 300 diffuses the aroma into the target space R, the change learning unit 650 stores the activity or the mood of the person in the target space R being identified by the identification unit 620 after a lapse of a predetermined time from the start of diffusion, as the activity or the mood of the person in the target space R after the aroma diffuser 300 diffuses the aroma into the target space R, in the change database 676 of the storage unit 670.

The change learning unit 650 performs learning suitable for evaluation on an effect of the aroma. For example, when it is identified that the mood of the person in the target space R is a drowsy state and the aroma diffuser 300 thereby diffuses, into the target space R, the aroma corresponding to the aroma pattern determined based on the mood, the change learning unit 650 learns about as to whether the mood of the person in the target space R is changed to an awakened state (i.e., as to whether the mood is not identified as the drowsy state). In addition, when it is identified that the mood of the person in the target space R is a grumpy state or a gloomy state and the aroma diffuser 300 thereby diffuses, into the target space R, the aroma corresponding to the aroma pattern determined based on the mood, the change learning unit 650 learns about as to whether the mood of the person in the target space R is changed to a pleasant state (i.e., as to whether the mood is not identified as the grumpy state or the gloomy state). In addition, it is identified that the activity of the person in the target space R is wake-up and the aroma diffuser 300 thereby diffuses, into the target space R, the aroma corresponding to the aroma pattern determined based on the activity, the change learning unit 650 learns about as to whether the mood of the person in the target space R is changed to an awakened state (i.e., as to whether the mood is not identified as the drowsy state) or as to whether the activity of the person in the target space R is changed to the working state.

The change learning unit 650 does not always necessarily learn about a change in at least one of the activity and the mood of the person in the target space R before and after the aroma diffuser 300 diffuses the aroma corresponding to the aroma pattern into the target space R. For example, the change learning unit 650 may be configured to learn only in a case where the activity or the mood of the person in the target space R, which the aroma determination unit 630 uses for determining the aroma pattern, is of a predetermined type.

The information which the change learning unit 650 learns about and the change database 676 of the storage unit 670 stores is displayed on, for example, the display unit (not illustrated) of the remote controller 30. The information which the change learning unit 650 learns about and the change database 676 of the storage unit 670 stores may also be transmitted to a mobile information terminal, a computer or the like of the user. The control apparatus 600 may provide to the user a recommendation about how the control should be changed being derived based on the information stored in the change database 676, instead of providing to the user the information itself stored in the change database 676. When the effect of a certain aroma pattern is unsatisfactory (e.g., when the drowsy state does not change although the aroma is diffused), the user may appropriately change the information stored in the pattern database 674 and the information stored in the correspondence information database 672, through the input unit 660 based on the provided information.

The change learning unit 650 may automatically change the information stored in the pattern database 674 and/or the information stored in the correspondence information database 672, based on the result of the learning.

For example, when the effect of a certain aroma pattern is unsatisfactory (e.g., when the drowsy state does not change although the aroma is diffused), the change learning unit 650 may appropriately rewrite the information stored in the pattern database 674 (e.g., an aroma type, an aroma diffusion amount, an aroma diffusion duration). Specifically, when the drowsiness of a person does not change although the aroma diffuser 300 diffuses the aroma, the change learning unit 650 may rewrite the information stored in the pattern database 674 such that the aroma diffuser 300 diffuses a more stimulating aroma or a stronger (denser) aroma.

For example, when the effect of a certain aroma pattern is unsatisfactory (e.g., when the drowsy state does not change although the aroma is diffused), the change learning unit 650 may appropriately rewrite the information stored in the correspondence information database 672. Specifically, when the drowsiness of a person does not change although the aroma diffuser 300 diffuses the aroma, the change learning unit 650 may rewrite the information stored in the correspondence information database 672 such that the aroma diffuser 300 diffuses, under the same conditions (the same person's activity or mood, the same temperature, the same humidity), a stronger aroma as compared with the aroma diffused before the change learning unit 650 rewrites the information.

### (2-3-6) Input Unit

The input unit 660 is a functional unit that receives an instruction on the aroma diffuser 300 from the user. For example, the input unit 660 receives an instruction to turn on or off the aroma diffuser 300 and an instruction to automatically or manually operate the aroma diffuser 300. In the aroma diffusion system 1, when the input unit 660 receives an instruction to automatically operate the aroma diffuser 300, the aroma determination unit 630 determines the aroma pattern of the aroma diffuser 300 based on a result of identification by the identification unit 620, and the aroma diffuser 300 diffuses the aroma corresponding to the determined aroma pattern into the target space R. A description on the operation of the aroma diffuser 300 to be manually operated will not be given here.

Preferably, in the control apparatus 600, information stored in the correspondence information database 672 and information stored in the pattern database 674 are configured to be rewritable based on user inputs to the input unit 660. In other words, preferably, in the control apparatus 600, the aroma pattern to be determined in accordance with the activity or the mood of the person in the target space R and the temperature and the humidity of the target space R is changeable by a user input to the input unit 660. Preferably, in the control apparatus 600, the aroma type, the aroma diffusion amount, and the aroma diffusion duration as to a certain aroma pattern are changeable by user inputs to the input unit 660.

Preferably, each of the correspondence information database 672 and the pattern database 674 stores default data in advance. FIG. 5 is a list of a combination of activities and moods of a person in the target space R with aromas typically suitable for the respective activities and moods. Each of the correspondence information database 672 and the pattern database 674 stores default data based on the information stored in FIG. 5. However, because there is a individual preference as to an aroma, the information in each of the correspondence information database 672 and the pattern database 674 is preferably changeable by an input through the input unit 660 as described above.

In the control apparatus 600, all the pieces of information in the correspondence information database 672 and pattern database 674 are not necessarily rewritable. In the control apparatus 600, for example, only the type of the aroma which the aroma diffuser 300 diffuses in accordance with a certain activity or a certain mood of a person in the target space R may be changeable by an input through the input unit 660.

### (2-3-7) Storage Unit

The storage unit 670 stores various kinds of information used in the control apparatus 600. For example, the storage unit 670 stores the image and the sound of the target space R acquired by the acquisition unit 610.

The storage unit 670 includes, as information storage regions, the correspondence information database 672, the pattern database 674, and the change database 676. The information stored in each of the correspondence information database 672, the pattern database 674, and the change database 676 has already been described above. Therefore, the description thereof will not be given here.

### (3) Aroma Control on Target Space

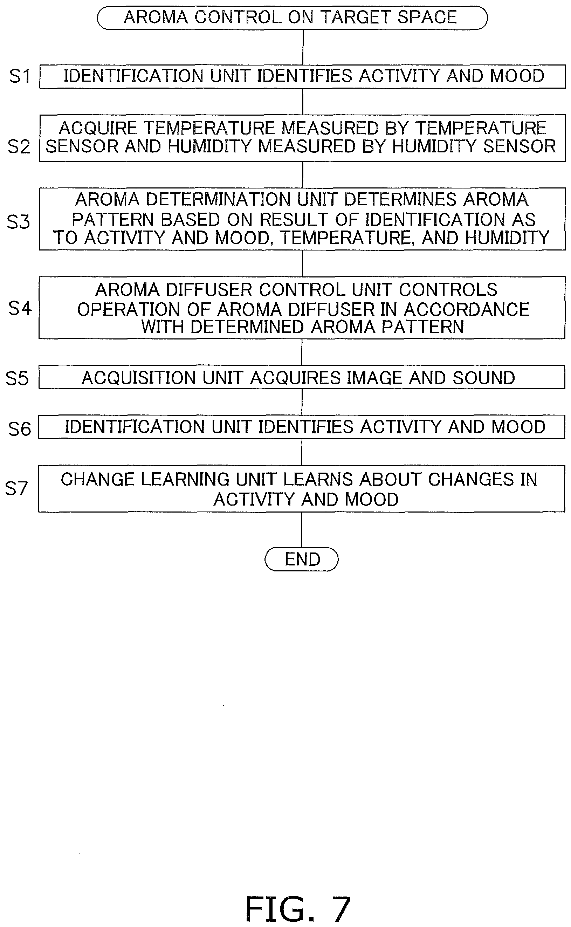

With reference to FIG. 7, a description will be given of aroma control on the target space R by the aroma diffusion system 1. The flowchart to be described below is merely an exemplary control flow, and therefore does not intend to limit a control flow.

It is assumed herein that the identification unit 620 identifies at least one of the activity and the mood of the person in the target space R based on person information acquired by the acquisition unit 610 every predetermined time (e.g., an image captured by the camera 410 and a sound acquired by the microphone 420 during a preceding predetermined period (e.g., 10 seconds)). The predetermined time may be a relatively shorter time (e.g., one minute) or may be a relatively longer time (e.g., 30 minutes). It is also assumed that the identification unit 620 identifies as "not applicable" when the person information acquired by the acquisition unit 610 does not apply to the activity and the mood of the person to be subjected to identification.

It is assumed that, at a certain time (i.e., in step S1), the identification unit 620 makes an identification, other than "not applicable", about the activity or the mood of the person in the target space R, based on the person information acquired by the acquisition unit 610. It is assumed herein that, for example, the identification unit 620 identifies the mood of the person in the target space R as the drowsy state. The identification unit 620 sends a result of the identification to the aroma determination unit 630.

When receiving the result of the identification from the identification unit 620, the aroma determination unit 630 acquires the temperature of the target space R measured by the temperature sensor 14 and the humidity of the target space R measured by the humidity sensor 16 (step S2).

Next, the aroma determination unit 630 determines the aroma pattern of the aroma diffuser 300 based on the activity or mood of the person in the target space R, the temperature of the target space R, and the humidity of the target space R (step S3). Specifically, the aroma determination unit 630 determines the aroma pattern of the aroma diffuser 300, based on the information stored in the storage unit 670 (i.e., the information in the correspondence information database 672, specifically the list illustrated in FIG. 4).

More specifically, the aroma determination unit 630 compares the temperature of the target space R with the threshold values to identify the level ("high", "middle" or "low" in the list illustrated in FIG. 4) of the temperature of the target space R. The aroma determination unit 630 also compares the humidity of the target space R with the threshold values to identify the level ("high", "middle" or "low" in the list illustrated in FIG. 4) of the humidity of the target space R. It is assumed herein that, for example, the aroma determination unit 630 identifies the temperature of target space R as the "middle" level in the list illustrated in FIG. 4, and also identifies the humidity of the target space R as the "middle" level in the list illustrated in FIG. 4. Next, the aroma determination unit 630 refers to the list illustrated in FIG. 4, ascertains a pattern (Pattern 5) corresponding to the activity or mood (the drowsy state) of the person in the target space R being identified by the identification unit 620, the level (the "middle" level) of the temperature of the target space R, and the level (the "middle" level) of the humidity of the target space R, and determines this pattern as the aroma pattern of the aroma diffuser 300.

In step S4, next, the aroma diffuser control unit 640 controls the operation of the aroma diffuser 300 in accordance with the aroma pattern determined by the aroma determination unit 630. Specifically, the aroma diffuser control unit 640 refers to the storage unit 670 (i.e., the pattern database 674, specifically the list illustrated in FIG. 6), and ascertains the aroma type, the aroma diffusion amount, and the aroma diffusion duration corresponding to the aroma pattern determined by the aroma determination unit 630. For example, in the case where the aroma pattern determined by the aroma determination unit 630 is "Pattern 5", the aroma diffuser control unit 640 refers to the list illustrated in FIG. 6, and ascertains the aroma type, the aroma diffusion amount, and the aroma diffusion duration as "herb-based", "b2", and "T1 ", respectively. The aroma diffuser control unit 640 controls the operation of the aroma diffuser 300 in accordance with the contents thus ascertained. In other words, the aroma diffuser control unit 640 controls the operation of the aroma diffuser 300 such that the aroma diffuser 300 diffuses the aroma of a "herb-based" in an amount "b2" per unit time for a duration "T1 ".

After a lapse of a predetermined time from the start of diffusion of the aroma from the aroma diffuser 300 under the control by the aroma diffuser control unit 640, the acquisition unit 610 acquires person information on the activity and mood of the person in the target space R (step S5). Specifically, the image acquisition unit 612 acquires image data (e.g., image data obtained for the preceding 10 seconds) of the target space R from the camera 410, and the sound acquisition unit 614 acquires sound data (e.g., sound data obtained for the preceding 10 seconds) of the target space R from the microphone 420. The acquisition unit 610 may acquire the person information during the diffusion of the aroma from the aroma diffuser 300. Alternatively, the acquisition unit 610 may acquire the person information after the diffusion of the aroma from the aroma diffuser 300 (e.g., after a lapse of the time "T1" from the start of diffusion of the aroma from the aroma diffuser 300).

In step S6, next, the identification unit 620 identifies at least one of the activity and the mood of the person in the target space R, based on the person information acquired by the acquisition unit 610.

In step S7, next, the change learning unit 650 learns about a change in at least one of the activity and the mood of the person before and after the aroma diffuser 300 diffuses the aroma corresponding to the aroma pattern. Specifically, the change learning unit 650 stores the aroma pattern determined in step S3 and the activity or mood before and after the aroma diffuser 300 diffuses the aroma (i.e., the result of the identification in step S1 and the result of the identification in step S6) in the change database 676 of the storage unit 670, with associating the aroma pattern with the activity or mood. The change learning unit 650 may appropriately rewrite the information in the correspondence information database 672 and the information in the pattern database 674 in accordance with a result of the learning, as described above.

After step S7, the identification unit 620 identifies the activity or mood of the person in the target space R again every predetermined time, based on the person information acquired by the acquisition unit 610. When the identification unit 620 makes an identification, other than "not applicable", about the activity or mood of the person in the target space R, based on the person information acquired by the acquisition unit 610, the series of processes in and subsequent to step S2 is carried out again.

### (4) Features

(4-1)

The aroma diffusion system 1 according to this embodiment of the invention includes the aroma diffuser 300 as an example of the aroma diffusion unit, the acquisition unit 610, the identification unit 620, and the aroma determination unit 630. The aroma diffuser 300 has a plurality of patterns of aromas to be diffused, and diffuses an aroma corresponding to the pattern of aroma into the target space R. The acquisition unit 610 acquires person information on at least one of the activity and the mood of a person in the target space R. The identification unit 620 identifies at least one of the activity and the mood of the person in the target space R, based on the person information acquired by the acquisition unit 610. The aroma determination unit 630 determines the pattern of the aroma of the aroma diffuser 300, based on a result of the identification by the identification unit 620.

According to this configuration, the aroma determination unit 630 determines the aroma to be diffused, based on the activity and/or the mood of a person. This configuration therefore enables diffusion of an aroma suitable for the person in the target space R.

(4-2)

In the aroma diffusion system 1 according to this embodiment of the invention, the identification unit 620 identifies multiple types of the activities and multiple types of the moods. The aroma diffusion system 1 further includes the storage unit 670 storing the multiple types of the activities, the multiple types of the moods, and the aroma patterns, with associating the respective activities and the respective moods with the aroma patterns. The aroma determination unit 630 determines the pattern of aroma based on the information stored in the storage unit 670.

Since the activities and/or the moods of the person, and the patterns of aromas are stored with associating the respective activities and/or the respective moods of the person with the aroma patterns, this configuration can diffuse a aroma suitable for the activity and/or the mood of the person.

(4-3)

In the aroma diffusion system 1 according to this embodiment, the acquisition unit 610 includes the image acquisition unit 612 that acquires the image of the target space R.

Preferably, the identification unit 620 identifies at least one of the activity and the mood of the person at least based on partial-body motion of the person in the target space R (a partial-body region image of the person) being acquired by the image acquisition unit 612.

Also preferably, in place of the partial-body motion of the person in the target space R being acquired by the image acquisition unit 612 or in addition to the partial-body motion of the person in the target space R being acquired by the image acquisition unit 612, the identification unit 620 identifies at least one of the activity and the mood of the person based on whole-body motion of the person in the target space R (a whole-body region image of the person) being acquired by the image acquisition unit 612.

According to this configuration, the identification unit 620 can accurately identifies at least one of the activity and the mood of the person based on the partial-body motion of the person (e.g., a change in facial expression, a movement of an arm or a leg) or the whole-body motion of the person.

(4-4)

In the aroma diffusion system 1 according to this embodiment, the acquisition unit 610 includes the sound acquisition unit 614 that acquires a sound which the person in the target space R makes.

Preferably, in place of the image of the target space R acquired by the image acquisition unit 612 or in addition to the image of the target space R acquired by the image acquisition unit 612, the identification unit 620 identifies at least one of the activity and the mood based on the sound which the person in the target space R makes being acquired by the sound acquisition unit 614.

According to this configuration, the identification unit 620 can accurately identify the activity and/or the mood of the person based on the sound which the person makes. In particular, in a case when the identification unit 620 identifies the activity and/or the mood of the person based on both the image and the sound, more accurate identification can be achieved as compared with the case where the identification unit 620 identifies the activity and/or the mood of the person based on either the image or the sound.

(4-5)

In the aroma diffusion system 1 according to this embodiment, the aroma diffuser 300 is disposed in the indoor unit 10 of the air conditioning apparatus 100. Therefore, it is possible to facilitate the spread of the aroma diffused from the aroma diffuser 300 with the indoor fan 12 of the indoor unit 10 throughout the target space R.

(4-6)

The aroma diffusion system 1 according to this embodiment of the invention includes at least one of the temperature sensor 14 that measures a temperature of the target space R and the humidity sensor 16 that measures a humidity of the target space R. More preferably, as shown in this embodiment, the aroma diffusion system 1 includes both the temperature sensor 14 and the humidity sensor 16. The aroma determination unit 630 determines the pattern of aroma based on the result of the measurement by the temperature sensor 14 and the result of the measurement by the humidity sensor 16. Each of the temperature sensor 14 and the humidity sensor 16 is an example of the measurement unit.

According to this configuration, an aroma to be diffused is determined, based on an environment of the target space R, in addition to the activity and the mood of a person. This configuration therefore enables diffusion of an aroma optimal for the person in the target space R.

(4-7)

The aroma diffusion system 1 according to this embodiment of the invention includes the change learning unit 650 that learns about a change in at least one of the activity and the mood before and after the aroma diffuser 300 diffuses the aroma corresponding to the pattern of aroma. The change learning unit 650 is an example of the learning unit.

According to this configuration, learning about changes in the activity and/or the mood of the person as a result of the diffusion of an aroma enables evaluation as to whether the aroma determination unit 630 has determined an appropriate aroma.

According to this configuration, the user is able to manually correct the details about the control of the aroma diffuser 300 through the input unit 660 while referring to the result of the learning by the change learning unit 650. According to this configuration, the control apparatus 600 is capable of automatically correcting the details about the control of the aroma diffuser 300, based on the result of the learning by the change learning unit 650. As a result, the aroma diffusion system 1 is particularly apt to optimize an aroma to be diffused in accordance with the activity and the mood of the person.

### (5) Modifications

Next, a description will be given of modifications of the foregoing embodiment. It should be noted that some of or all of the configurations in the respective modifications may be combined with some of or all of the configurations in the other modifications insofar as there are no consistencies.

### (5-1) Modification A

In the foregoing embodiment, the control apparatus 600 of the aroma diffusion system 1 includes the controller 50 of the air conditioning apparatus 100 and the server 200. However, the control apparatus 600 is not limited to this configuration. For example, the aroma diffusion system 1 may not include the server 200, and only the controller 50 may function as the control apparatus 600. Alternatively, the server 200 may mainly function as the control apparatus 600 and control the aroma diffuser 300 in such a manner that the control apparatus 600 transmits an instruction to the aroma diffuser 300.

### (5-2) Modification B

In the foregoing embodiment, the single aroma diffuser 300, which is an example of the aroma diffusion unit, diffuses the aroma corresponding to the pattern into the target space R. However, the configuration of the aroma diffusion unit is not limited thereto.

For example, the aroma diffusion unit may include a plurality of aroma diffusers that respectively diffuse aromas different in type from one another. The aroma diffuser control unit 640 may control one or more aroma diffusers in accordance with one or more aroma types determined by the aroma determination unit 630.

### (5-3) Modification C

In the foregoing embodiment, the identification unit 620 identifies both the activity and the mood of the person in the target space R. However, the configuration of the identification unit 620 is not limited thereto. For example, the identification unit 620 may identify only one of the activity and the mood of the person in the target space R.

### (5-4) Modification D

In the foregoing embodiment, the aroma diffusion system 1 includes the camera 410 and the microphone 420. However, the configuration of the aroma diffusion system 1 is not limited to thereto. The aroma diffusion system 1 may acquire an image captured by a camera provided separately from the system (e.g., a mobile information terminal of the user) and a sound collected by a microphone provided separately from the system (e.g., the mobile information terminal of the user).

### (5-5) Modification E

In the foregoing embodiment, the aroma pattern of the aroma diffuser 300 is determined based on a result of identification by the identification unit 620, and the aroma diffuser 300 diffuses the aroma corresponding to the determined aroma pattern into the target space R. In addition to this aspect, the aroma diffusion system 1 may perform as follows.

For example, in a case where the identification unit 620 identifies the activity of the person in the target space R as a specific activity and in a case where the identification unit 620 identifies the mood of the person in the target space R as a specific mood, the aroma diffusion system 1 may stop the operation of the aroma diffuser 300. That is, in a case where the identification unit 620 identifies an activity of a person in the target space R as a specific activity and in a case where the identification unit 620 identifies a mood of the person in the target space R as a specific mood, the aroma diffusion system 1 may stop the operation of the aroma diffuser 300.

### (5-6) Modification F

In the foregoing embodiment, the control apparatus 600 of the aroma diffusion system 1 includes the controller 50 of the air conditioning apparatus 100 and the server 200. However, the configuration of the control apparatus 600 is not limited thereto. For example, the aroma diffuser 300 may include a controller, and the control apparatus 600 of the aroma diffusion system 1 may include the controller of the aroma diffuser 300 and the server 200.

### (5-7) Modification G

In the foregoing embodiment, the identification unit 620 identifies the activity and the mood of the person in the target space R, using the discriminator 626 learned based on machine learning. However, the identification unit 620 does not necessarily identify the activity and the mood of the person in the target space R using the discriminator 626. For example, when it is possible to manually give a rule (e.g., a rule that when an image or a sound has a feature α, a person in the target space R is in a state of an activity β) to the control apparatus 600 in advance, the identification unit 620 may identify the activity and the mood of the person in the target space R based on this rule. The rule to be given herein is, for example, a rule that a person in the target space R is grumpy if he or she holds his or her arms while knitting his or her eyebrows.

### (5-8) Modification H

In the foregoing embodiment, in the case of using an image for its identification, the identification unit 620 identifies the activity and/or the mood of the person in the target space R based on at least one of the person whole-body region image and the person partial-body region image (i.e., based on an image of the person). However, the identification method is not limited thereto. For example, the identification unit 620 may identify the activity and the mood of the person in the target space R based on, in addition to the image of the person, information on a background image (e.g., information about where the person is).

### (5-9) Modification I

In the foregoing embodiment, the aroma diffuser 300 is disposed in the indoor unit 10 of the air conditioning apparatus 100. However, the configuration of the aroma diffuser 300 is not limited thereto. The aroma diffuser 300 may alternatively be disposed in another apparatus such as an air cleaner or a humidifier. Alternatively, the aroma diffuser 300 may be an independent apparatus (i.e., an apparatus installed in the target space R independently of the air conditioning apparatus 100 and the like).

### INDUSTRIAL APPLICABILITY

The present disclosure is widely applicable to and useful for an aroma diffusion system having a plurality of patterns of aromas to be diffused.

### REFERENCE SIGNS LIST

1: aroma diffusion system
10: indoor unit
14: temperature sensor (measurement unit)
16: humidity sensor (measurement unit)
100: air conditioning apparatus
300: aroma diffuser (aroma diffusion unit)
610: acquisition unit
612: image acquisition unit
614: sound acquisition unit
620: identification unit
630: aroma determination unit
650: change learning unit (learning unit)
670: storage unit
R: target space

### CITATION LIST

### PATENT LITERATURE

Patent Literature 1: JP 2007-170761 A

## Claims

1. An aroma diffusion system (1) comprising:
an aroma diffusion unit (300) having a plurality of aroma patterns to be diffused, the aroma diffusion unit (300) being configured to diffuse an aroma corresponding to the aroma pattern into a target space (R);
an acquisition unit (610) configured to acquire person information on at least one of an activity and a mood of a person in the target space;
a measurement unit (14, 16) configured to measure a temperature and a humidity of the target space;
a storage unit (670) including, as information storage regions, a correspondence information database (672) and a pattern database (674),
the correspondence information database storing a list of a combination of the activity or the mood of the person in the target space, the temperature of the target space, and the humidity of the target space, and the aroma pattern, the combination and the aroma pattern being associated with each other in the list,
the pattern database storing a list of an aroma type, an aroma diffusion amount, and an aroma diffusion duration set for each of the aroma patterns;
and
an identification unit (620) configured to identify at least one of the activity and the mood of the person in the target space, based on the person information acquired by the acquisition unit;
an aroma determination unit (630) configured to determine the aroma pattern of the aroma diffusion unit based on the information stored in the correspondence information database, a result of the identification by the identification unit, and the temperature of the target space and the humidity of the target space;
an aroma diffuser control unit (640) configured to, when the aroma determination unit determines the aroma pattern, read, referring to the pattern database, the aroma type, the aroma diffusion amount, and the aroma diffusion duration corresponding to the aroma pattern determined by the aroma determination unit, and control the aroma diffuser to operate under the read-out conditions to diffuse an aroma corresponding to the determined aroma pattern into the target space,
**characterized by**:
a learning unit (650) configured to learn about a change in at least one of the activity and the mood before and after the aroma diffusion unit diffuses the aroma corresponding to the aroma pattern, and rewrite at least one of the information in the correspondence information database and the information in the pattern database in accordance with the result of the learning.

2. The aroma diffusion system according to claim 1, wherein
the activity to be identified by the identification unit includes at least one of a rising state, a sleeping state and a working state.

3. The aroma diffusion system according to claim 1 or 2, wherein
the mood to be identified by the identification unit includes at least one of a drowsy state, a tired state, a grumpy state and a gloomy state.

4. The aroma diffusion system according to any one of claims 1 to 3, wherein
the acquisition unit includes an image acquisition unit (612) configured to acquire an image of the target space, and
the identification unit is configured to identify at least one of the activity and the mood at least based on partial-body motion of the person in the target space, the partial-body motion being acquired by the image acquisition unit.

5. The aroma diffusion system according to any one of claims 1 to 3, wherein
the acquisition unit includes an image acquisition unit (612) configured to acquire an image of the target space, and
the identification unit is configured to identify at least one of the activity and the mood at least based on whole-body motion of the person in the target space, the whole-body motion being acquired by the image acquisition unit.

6. The aroma diffusion system according to any one of claims 1 to 5, wherein
the acquisition unit includes a sound acquisition unit (614) configured to acquires a sound which the person in the target space makes, and
the identification unit is configured to identify at least one of the activity and the mood at least based on the sound which the person in the target space makes, the sound being acquired by the sound acquisition unit.

7. The aroma diffusion system according to any one of claims 1 to 6, wherein
the aroma diffusion unit is disposed in an indoor unit (10) of an air conditioning apparatus (100).

## Patentansprüche

1. Aromadiffusionssystem (1), das aufweist:
eine Aromadiffusionseinheit (300) mit mehreren zu diffundierenden Aromamustern, wobei die Aromadiffusionseinheit (300) konfiguriert ist, ein dem Aromamuster entsprechendes Aroma in einen Zielraum (R) zu diffundieren;
eine Erfassungseinheit (610), die konfiguriert ist, Personeninformationen über mindestens eine Aktivität und eine Stimmung einer Person im Zielraum zu erfassen;
eine Messeinheit (14, 16), die konfiguriert ist, eine Temperatur und eine Feuchtigkeit des Zielraums zu messen;
eine Speichereinheit (670), die als Informationsspeicherbereiche eine Korrespondenzinformationsdatenbank (672) und eine Musterdatenbank (674) enthält,
wobei die Korrespondenzinformationsdatenbank eine Liste einer Kombination der Aktivität oder der Stimmung der Person im Zielraum, der Temperatur des Zielraums und der Feuchtigkeit des Zielraums und des Aromamusters speichert, wobei die Kombination und das Aromamuster in der Liste miteinander verknüpft sind,
die Musterdatenbank eine Liste eines Aromatyps, einer Aromadiffusionsmenge und einer für jedes der Aromamuster eingestellten Aromadiffusionsdauer speichert; und
eine Identifizierungseinheit (620), die konfiguriert ist, mindestens eine der Aktivität und der Stimmung der Person im Zielraum basierend auf den Personeninformationen zu identifizieren, die durch die Erfassungseinheit erfasst werden;
eine Aromabestimmungseinheit (630), die konfiguriert ist, das Aromamuster der Aromadiffusionseinheit basierend auf den in der Korrespondenzinformationsdatenbank gespeicherten Informationen, einem Ergebnis der Identifikation durch die Identifikationseinheit und der Temperatur des Zielraums und der Feuchtigkeit des Zielraums zu bestimmen;
eine Aromadiffusor-Steuereinheit (640), die konfiguriert ist, wenn die Aromabestimmungseinheit das Aromamuster bestimmt, unter Bezugnahme auf die Musterdatenbank den Aromatyp, die Aromadiffusionsmenge und die Aromadiffusionsdauer, die dem von der Aromabestimmungseinheit bestimmten Aromamuster entspricht, auszulesen und den Aromadiffusor zu steuern, gemäß den ausgelesenen Bedingungen zu arbeiten, um ein dem bestimmten Aromamuster entsprechendes Aroma in den Zielraum zu diffundieren,
**gekennzeichnet durch**:
eine Lerneinheit (650), die konfiguriert ist, etwas über eine Änderung mindestens einer der Aktivität und der Stimmung zu lernen, bevor und nachdem die Aromadiffusionseinheit das dem Aromamuster entsprechende Aroma diffundiert, und mindestens einer der Informationen in der Korrespondenzinformationsdatenbank und der Informationen in der Musterdatenbank gemäß dem Ergebnis des Lernens neu zu schreiben.

2. Aromadiffusionssystem nach Anspruch 1, wobei die durch die Identifikationseinheit zu identifizierende Aktivität mindestens einen von einem Aufstehzustand, einem Schlafzustand und einem Arbeitszustand aufweist.

3. Aromadiffusionssystem nach Anspruch 1 oder 2, wobei die von der Identifizierungseinheit zu identifizierende Stimmung mindestens einen von einem schläfrigen Zustand, einem müden Zustand, einem mürrischen Zustand und einem düsteren Zustand aufweist.

4. Aromadiffusionssystem nach einem der Ansprüche 1 bis 3, wobei
die Erfassungseinheit eine Bilderfassungseinheit (612) aufweist, die konfiguriert ist, ein Bild des Zielraums zu erfassen, und
die Identifizierungseinheit konfiguriert ist, mindestens eine der Aktivität und der Stimmung mindestens basierend auf der Teilkörperbewegung der Person im Zielraum zu identifizieren, wobei die Teilkörperbewegung durch die Bilderfassungseinheit erfasst wird.

5. Aromadiffusionssystem nach einem der Ansprüche 1 bis 3, wobei
die Erfassungseinheit eine Bilderfassungseinheit (612) aufweist, die konfiguriert ist, ein Bild des Zielraums zu erfassen, und
die Identifizierungseinheit konfiguriert ist, mindestens eine der Aktivität und der Stimmung mindestens basierend auf der Ganzkörperbewegung der Person im Zielraum zu identifizieren, wobei die Ganzkörperbewegung von der Bilderfassungseinheit erfasst wird.

6. Aromadiffusionssystem nach einem der Ansprüche 1 bis 5, wobei
die Erfassungseinheit eine Geräuscherfassungseinheit (614) enthält, die konfiguriert ist, ein Geräusch zu erfassen, das die Person im Zielraum erzeugt, und
die Identifizierungseinheit konfiguriert ist, mindestens eine der Aktivität und der Stimmung mindestens basierend auf dem Geräusch zu identifizieren, das die Person in dem Zielraum erzeugt, wobei das Geräusch von der Geräuscherfassungseinheit erfasst wird.

7. Aromadiffusionssystem nach einem der Ansprüche 1 bis 6, wobei die Aromadiffusionseinheit in einer Inneneinheit (10) einer Klimatisierungsvorrichtung (100) angeordnet ist.

## Revendications

1. Système de diffusion d'arômes (1), comprenant :
une unité de diffusion d'arômes (300) ayant une pluralité de motifs d'arômes à diffuser, ladite unité de diffusion d'arômes (300) étant prévue pour diffuser un arôme correspondant au motif d'arômes dans un espace de destination (R) ;
une unité d'acquisition (610) prévue pour acquérir des informations de personne sur une activité et/ou un état d'esprit d'une personne dans l'espace de destination ;
une unité de mesure (14, 16) prévue pour mesurer une température et une humidité de l'espace de destination ; une unité de stockage (670) contenant, en tant que zones de stockage d'informations, une base de données d'informations de correspondance (672) et une base de données de motifs (674),
ladite base de données d'informations de correspondance stockant une liste de combinaison de l'activité ou de l'état d'esprit de la personne dans l'espace de destination, la température de l'espace de destination, l'humidité de l'espace de destination et le motif d'arômes, la combinaison et le motif d'arômes étant associés l'une à l'autre dans la liste,
la base de données de motifs stockant une liste d'un type d'arôme, d'une valeur de diffusion d'arôme et d'une durée de diffusion d'arôme fixée pour chacun des motifs d'arômes ; et
une unité d'identification (620) prévue pour identifier l'activité et/ou l'état d'esprit de la personne dans l'espace de destination, sur la base des informations de personne acquises par l'unité d'acquisition ;
une unité de détermination d'arômes (630) prévue pour déterminer le motif d'arômes de l'unité de diffusion d'arômes sur la base des informations stockées dans la base de données d'informations de correspondance, d'un résultat d'identification par l'unité d'identification, de la température de l'espace de destination et de l'humidité de l'espace de destination ;
une unité de commande de diffuseur d'arômes (640) prévue pour extraire en référence à la base de données de motifs, lorsque l'unité de détermination d'arômes détermine le motif d'arômes, le type d'arôme, la valeur de diffusion d'arôme et la durée de diffusion d'arôme correspondant au motif d'arômes déterminé par l'unité de détermination d'arômes, et commander le fonctionnement du diffuseur d'arômes dans les conditions d'extraction pour diffuser un arôme correspondant au motif d'arômes déterminé dans l'espace de destination,
**caractérisé par** :
une unité d'apprentissage (650) prévue pour appréhender un changement dans l'activité et/ou l'état d'esprit avant et après diffusion par l'unité de diffusion d'arômes de l'arôme correspondant au motif d'arômes, et réécrire au moins une des informations dans la base de données d'informations de correspondance et des informations dans la base de données de motifs en fonction du résultat d'apprentissage.

2. Système de diffusion d'arômes selon la revendication 1, où l'activité à identifier par l'unité d'identification comprend un état de réveil et/ou un état de sommeil et/ou un état d'activité.

3. Système de diffusion d'arômes selon la revendication 1 ou la revendication 2, où
l'état d'esprit à identifier par l'unité d'identification comprend un état de somnolence et/ou un état de fatigue et/ou un état de mauvaise humeur et/ou un état morose.

4. Système de diffusion d'arômes selon l'une des revendications 1 à 3, où
l'unité d'acquisition comprend une unité d'acquisition d'image (612) prévue pour acquérir une image de l'espace de destination, et
l'unité d'identification est prévue pour identifier l'activité et/ou l'état d'esprit au moins sur la base d'un mouvement corporel partiel de la personne dans l'espace de destination, ledit mouvement corporel partiel étant acquis par l'unité d'acquisition d'image.

5. Système de diffusion d'arômes selon l'une des revendications 1 à 3, où
l'unité d'acquisition comprend une unité d'acquisition d'image (612) prévue pour acquérir une image de l'espace de destination, et
l'unité d'identification est prévue pour identifier l'activité et/ou l'état d'esprit au moins sur la base d'un mouvement corporel intégral de la personne dans l'espace de destination, ledit mouvement corporel intégral étant acquis par l'unité d'acquisition d'image.

6. Système de diffusion d'arômes selon l'une des revendications 1 à 5, où
l'unité d'acquisition comprend une unité d'acquisition de son (614) prévue pour acquérir un son émis par la personne dans l'espace de destination, et
l'unité d'identification est prévue pour identifier l'activité et/ou l'état d'esprit au moins sur la base du son émis par la personne dans l'espace de destination, ledit son étant acquis par l'unité d'acquisition de son.

7. Système de diffusion d'arômes selon l'une des revendications 1 à 6, où l'unité de diffusion d'arômes est disposée dans une unité intérieure (10) d'un appareil de climatisation (100).
